# EUROPEAN PATENT APPLICATION

(11) **EP 4 729 167 A1**
(43) Date of publication of application: **22.04.2026**
(21) Application number: 24873072.3
(22) Date of filing: 27.09.2024
(51) Int. Cl.: B01J 19/00, C07K 1/04

(54) **FLOW REACTOR AND BIOLOGICAL POLYMER SYNTHESIS SYSTEM INCLUDING SAME**

(30) Priority: 27.09.2023 KR 20230131229
(71) Applicant: SP2TX Inc., Seoul 08826 (KR)
(72) Inventor: CHOI, Jae Sun, Seoul 08813 (KR); CHOI, Min Hyeok, Seoul 08848 (KR)
(74) Representative: Appleyard Lees IP LLP
(86) International application number: PCT/KR2024/014797
(87) International publication number: WO 2025/071365

(57) **Abstract**

The present disclosure relates to a flow reactor and a biopolymer synthesis system including the same. The biopolymer synthesis system includes: a mixing reservoir in which at least one of an amino acid, a reagent, and an additive is mixed; a reactor into which a fluid discharged from the mixing reservoir is introduced; a temperature controller arranged between the mixing reservoir and the reactor and configured to control a temperature of the fluid introduced into the reactor; and a pump configured to provide a driving force such that the fluid flows from the mixing reservoir to the reactor, wherein the reactor includes: a column having an inlet through which the fluid enters and an outlet through which the fluid flows out; and a composite solid support arranged inside the column.

## Description

### Technical Field

The present disclosure relates to a flow reactor and a biopolymer synthesis system.

### Background Art

Peptide medicines generate billions of dollars in annual sales in the fields of diabetes, obesity, and oncology industries, and the scope of peptide medicines is expanding with the development of new drugs for emerging diseases such as cardiovascular and neurodegenerative diseases and the like. Currently, the disadvantage of peptides, i.e., short half-life in the body, has been solved through methods such as introduction of unnatural amino acids. Various peptide medicines with dosing cycles of at least one week are already commercially available on the market, and many oral administration candidates are undergoing clinical trials with the U.S. Food and Drug Administration. Accordingly, demand for producing peptide medicines is increasing by nearly 10 % every year, and thus there is an urgent need for improvements in productivity to meet this demand and new synthesis platforms to address ESG issues such as restrictions on use of DMF and the like used in traditional chemical synthesis methods.

Peptide medicines are manufactured largely by biological methods or chemical methods. Biological methods using genetically modified or transgenic plants and animals have the disadvantage of being difficult to produce peptide medicines with extended half-life using unnatural amino acids or PEGylation which reflect recent trends, and the disadvantage of being difficult to remove various endogenous contaminants derived from organisms or exogenous contaminants generated during a manufacturing process.

In chemical methods, liquid-phase reactions require low process costs, but only peptides consisting of 10 or fewer amino acids can be synthesized, and thus it is generally impossible to produce peptide medicines consisting of 20 or more amino acids. In this regard, a solid-phase peptide synthesis method (hereinafter referred to as SPPS) is the most suitable method for manufacturing PEGylated peptide medicines consisting of 20 to 50 different types of natural/unnatural amino acids.

To enhance production capacity for synthesis of various biopolymers including peptides, various types of reactors are being studied in the SPPS field. Representative reactors include batch-type reactors and flow-type reactors. For large-scale synthesis, batch-type reactors have gradually scaled up from 10 to 50 mL to 100 to 500 L units currently. For this, improvements were made to various solution transfer, stirring, pressure control, and filtration methods. At the laboratory scale, flow reactors have been recently introduced and demonstrated potential for rapid peptide synthesis with high purity and high yield.

However, such batch-type reactors have drawbacks that the reactor size varies with reaction scales, leading to differences in heat transfer efficiency and time required for uniform mixing between the central portion and surface portion of a reaction solution, and that the reaction temperature/mixing reproducibility is low, leading to challenges in yield and quality control due to reproducibility issues.

On the other hand, typical flow-type reactors have the following advantages: consistent heat transfer based on increased reactive specific surface area resulting from high-speed mixing occurring by passing through a tube-shaped reactor; excellent reaction reproducibility through induced chemical reactions; and ease of quality control and yield management due to high reaction reproducibility.

Currently developed flow-type reactors have the advantage of rapid synthesis reaction progression, but when using 200 mg of PS/DVB, issues occur with a back pressure of 30 to 50 bar. As such, high back pressure clearly demonstrates that the synthesis scale cannot be increased significantly. In addition, the flow-type reactor developed by Vapourtek in the UK has overcome the high back pressure, which is a shortcoming of packed bead-type resins, by using an adjustable syringe. However, the available flow rate is very low, and due to the nature of pressure control using a syringe, it is difficult to significantly increase the synthesis scale.

In addition, existing particulate resins cannot be self-standing due to the limitations of the particulate structure, and are structurally limited in that the particulate resins are transported and packed by flow, leading to increased back pressure within a flow reactor.

Accordingly, a solid support material suitable for flow reactors for the SPPS and capable of self-standing and a biopolymer synthesis system using the solid support material have been newly developed.

### Disclosure of Invention

### Technical Problem

The present disclosure aims to provide: a flow reactor suitable for SPPS, having enhanced synthesis efficiency and yields and high durability and stability; and a biopolymer synthesis system including the same.

### Solution to Problem

An embodiment of the present disclosure provides a biopolymer synthesis system including: a mixing reservoir in which at least one of an amino acid, a reagent, and an additive is mixed; a reactor into which a fluid discharged from the mixing reservoir is introduced; a temperature controller arranged between the mixing reservoir and the reactor and configured to control a temperature of the fluid introduced into the reactor; and a pump configured to provide a driving force such that the fluid flows from the mixing reservoir to the reactor, wherein the reactor includes: a column having an inlet through which the fluid enters and an outlet through which the fluid flows out; and a composite solid support arranged inside the column.

### Advantageous Effects of Disclosure

A flow reactor and a biopolymer synthesis system including the same, according to the present disclosure, may provide a biopolymer synthesis process with enhanced efficiency and yields. Specifically, the biopolymer synthesis system utilizes a flow reactor provided with a composite solid support, demonstrating low pressure drop even at high flow velocity and exhibiting characteristics of maintaining excellent efficiency and yields across a wide range of fluid temperatures.

In addition, the flow reactor and the biopolymer synthesis system including the same, according to the present disclosure, may provide a device with improved durability and stability overall and enable precise process control.

### Brief Description of Drawings

FIG. 1 is a diagram illustrating a biopolymer synthesis system according to an embodiment of the present disclosure.
FIG. 2 is a drawing illustrating the reactor in FIG. 1.
FIG. 3 is a graph measuring pressure drop according to flow velocity, to compare the reactor of the present disclosure with a reactor of a comparative example.
FIG. 4 is a graph measuring pressure drop according to the size of the reactor of the present disclosure.
FIGS. 5 and 6 are graphs measuring temperature-dependent pressure drop in the reactor of the present disclosure and reactors of comparative examples.
FIG. 7 shows appearance of a core structure before undergoing coating, and FIG. 8 shows appearance of a composite solid support.
FIGS. 9 to 11 show HPLC graphs for synthesized ACP according to flow rate, by using a composite solid support according to an embodiment.

### Best Mode for the Invention

An embodiment of the present disclosure provides a biopolymer synthesis system including: a mixing reservoir in which at least one of an amino acid, a reagent, and an additive is mixed; a reactor into which a fluid discharged from the mixing reservoir is introduced; a temperature controller arranged between the mixing reservoir and the reactor and configured to control a temperature of the fluid introduced into the reactor; and a pump configured to provide a driving force such that the fluid flows from the mixing reservoir to the reactor, wherein the reactor includes: a column having an inlet through which the fluid enters and an outlet through which the fluid flows out; and a composite solid support arranged inside the column.

In addition, the composite solid support may include a core structure and a functional coating positioned on the core structure.

In addition, the composite solid support may be self-standing when the fluid passes through the column.

In addition, the functional coating in the composite solid support may have a loading density of 0.01 mmol/g to 2 mmol/g.

In addition, when the fluid passes through the reactor, a pressure drop between the inlet and outlet may be 10 bar or less at a flow velocity of 40 C.V/min or less. Specifically, the pressure drop may be measured based on the pressure difference occurring between the inlet and outlet of an empty column. In addition, the flow velocity may be expressed as column volume (C.V) per minute, and the reactor may have a pressure drop of 10 bar or less at a flow velocity of 40 C.V/min or less, specifically, 40 C.V/min or less, 30 C.V/min or less, 20 C.V/min or less, 0.1 C.V/min to 40 C.V/min, 1 C.V/min to 40 C.V/min, 5 C.V/min to 40 C.V/min, 10 C.V/min to 40 C.V/min, 15 C.V/min to 40 C.V/min, 20 C.V/min to 40 C.V/min, 25 C.V/min to 40 C.V/min, 30 C.V/min to 40 C.V/min, 35 C.V/min to 40 C.V/min, 0.1 C.V/min to 30 C.V/min, 1 C.V/min to 30 C.V/min, 5 C.V/min to 30 C.V/min, 10 C.V/min to 30 C.V/min, 15 C.V/min to 30 C.V/min, 20 C.V/min to 30 C.V/min, 25 C.V/min to 30 C.V/min, 0.1 C.V/min to 20 C.V/min, 1 C.V/min to 20 C.V/min, 5 C.V/min to 20 C.V/min, 10 C.V/min to 20 C.V/min, 15 C.V/min to 20 C.V/min, 0.1 C.V/min to 10 C.V/min, 1 C.V/min to 10 C.V/min, or 5 C.V/min to 10 C.V/min. Furthermore, within the aforementioned flow velocity ranges, the pressure drop may be 10 bar or less, specifically 10 bar or less, 5 bar or less, 3 bar or less, 1 bar or less, 0.5 bar or less, 0.1 bar or less, 0.05 bar or less, 0.01 bar to 10 bar, 0.05 bar to 10 bar, 0.1 bar to 10 bar, 0.5 bar to 10 bar, 1 bar to 10 bar, 3 bar to 10 bar, 5 bar to 10 bar, 0.01 bar to 5 bar, 0.05 bar to 5 bar, 0.1 bar to 5 bar, 0.5 bar to 5 bar, 1 bar to 5 bar, 3 bar to 5 bar, 0.01 bar to 3 bar, 0.05 bar to 3 bar, 0.1 bar to 3 bar, 0.5 bar to 3 bar, 1 bar to 3 bar, 0.01 bar to 1 bar, 0.05 bar to 1 bar, 0.1 bar to 1 bar, 0.5 bar to 1 bar, 0.01 bar to 0.5 bar, 0.05 bar to 0.5 bar, 0.1 bar to 0.5 bar, 0.01 bar to 0.1 bar, or 0.05 bar to 0.1 bar.

In addition, the temperature of the fluid that has passed through the temperature controller and flows into the reactor may be 120 °C or lower. The biopolymer system of the present disclosure may enable biopolymer synthesis with excellent purity and efficiency even in a wide temperature range of the fluid, and the temperature of the fluid may be, although not limited thereto, 120 °C or lower, specifically, 120 °C or lower, 100 °C or lower, 80 °C or lower, 60 °C or lower, 25 °C to 120 °C, 50 °C to 120 °C, 70 °C to 120 °C, 100 °C to 120 °C, 25 °C to 100 °C, 50 °C to 100 °C, 70 °C to 100 °C, 25 °C to 70 °C, 50 °C to 70 °C, or 25 °C to 50 °C. The temperature may be set without limitation if it is the temperature of the fluid used in the biopolymer synthesis.

In addition, the biopolymer synthesis system may further include a valve arranged between the reactor and the mixing reservoir and configured to discharge waste.

In addition, the biopolymer synthesis system may further include: a main line connecting the mixing reservoir, the pump, the temperature controller, and the reactor together; and a first branch line branching off from the main line between the mixing reservoir and the reactor and being connected to the mixing reservoir.

In addition, the fluid may have a circulating flow through the main line.

In addition, the biopolymer synthesis system may further include a solvent reservoir arranged on the first branch line.

In addition, the solvent reservoir may be connected to the valve and the mixing reservoir through the first branch line.

In addition, the biopolymer synthesis system may further include a sensor arranged on the main line and configured to sense at least one of a temperature, a pressure, and a flow rate of the fluid flowing along the main line.

In addition, the biopolymer synthesis system may further include a detector arranged on the main line and configured to detect progress of biopolymer synthesis.

Furthermore, the biopolymer synthesis system may further include a second branch line branching off from the valve and being configured to discharge the waste.

### Mode for the Invention

As the disclosure allows for various changes and numerous embodiments, exemplary embodiments will be illustrated in the drawings and described in detail in the written description. An effect and a characteristic of the present disclosure, and a method of accomplishing these will be apparent when referring to embodiments described with reference to the drawings. The present disclosure may, however, be embodied in many different forms and should not be construed as limited to the embodiments set forth herein.

Hereinafter, embodiments of the disclosure will be described in detail with reference to the accompanying drawings. The same or corresponding components will be denoted by the same reference numerals, and thus redundant description thereof will be omitted.

It will be understood that although the terms "first," "second," etc. may be used herein to describe various components, these components should not be limited by these terms. These components are only used to distinguish one component from another.

An expression used in the singular encompasses the expression of the plural, unless it has a clearly different meaning in the context.

It will be further understood that the terms "comprises" and/or "comprising" used herein specify the presence of stated features or elements, but do not preclude the presence or addition of one or more other features or elements. It will be understood that when a layer, region, or component is referred to as being "on" or "onto" another layer, region, or component, it may be directly or indirectly formed on the other layer, region, or component. That is, for example, intervening layers, regions, or components may be present.

Sizes of elements in the drawings may be exaggerated for convenience of explanation. In other words, since the sizes and thicknesses of components in the drawings are arbitrarily illustrated for convenience of explanation, the following embodiments are not limited thereto.

Hereinafter, it will be understood that when a layer, region, or component is referred to as being "connected to" another layer, region, or component, the layer, region, or component may be not only directly connected to the other layer, region, or component, but also indirectly connected to the other layer, region, or component as intervening layer, region, or component is present. For example, it will be understood that when a layer, region, or component is referred to as being "electrically connected to" another layer, region, or component, the layer, region, or component may be not only directly electrically connected to the other layer, region, or component, but also indirectly electrically connected to the other layer, region, or component as intervening layer, region, or component is present. Hereinafter, examples of the present disclosure will be described in detail with reference to the attached drawings.

In the present specification, the term "loading density" refers to "the number of moles of functional groups provided per unit mass of a composite solid support," and may refer to a reaction site for biopolymer synthesis according to a compositional ratio of a polymer for biopolymer synthesis according to an embodiment.

In an embodiment, a composite solid support may include: a core structure having a length of 1 mm or more and a shape of one dimension or higher dimension; and a functional coating surrounding the core structure, wherein a biopolymer may be synthesized on the functional coating.

In an embodiment, the functional coating may have a property of swelling in a solvent.

In an embodiment, the functional coating may include functional groups within or on the surface of the functional coating. Specifically, the functional coating may include functional groups, and due to the property of swelling in a solvent, reactants may easily penetrate into the functional coating by the concentration gradient. Accordingly, the functional groups within or on the surface of the functional coating may be easily exposed to the reactants, exhibiting high synthesis efficiency even at low loading density.

In an embodiment, the functional groups may include at least one selected from an amine group, a carboxyl group, a hydroxyl group, a carbonyl group, an amino group, a thiol group, and a phosphoric acid group.

In an embodiment, a loading density of the functional coating at a reaction site may be 0.01 mmol/g to 2 mmol/g. Specifically, the loading density of the functional coating at a reaction site may be 0.01 mmol/g to 2 mmol/g, 0.05 mmol/g to 2 mmol/g, 0.1 mmol/g to 2 mmol/g, 0.3 mmol/g to 2 mmol/g, 0.5 mmol/g to 2 mmol/g, 0.7 mmol/g to 2 mmol/g, 1 mmol/g to 2 mmol/g, 0.01 mmol/g to 1.7 mmol/g, 0.05 mmol/g to 1.7 mmol/g, 0.1 mmol/g to 1.7 mmol/g, 0.3 mmol/g to 1.7 mmol/g, 0.5 mmol/g to 1.7 mmol/g, 0.7 mmol/g to 1.7 mmol/g, 1.0 mmol/g to 1.7 mmol/g, 0.01 mmol/g to 1.5 mmol/g, 0.05 mmol/g to 1.5 mmol/g, 0.1 mmol/g to 1.5 mmol/g, 0.3 mmol/g to 1.5 mmol/g, 0.5 mmol/g to 1.5 mmol/g, 0.7 mmol/g to 1.5 mmol/g, 1.0 mmol/g to 1.5 mmol/g, 0.01 mmol/g to 1.2 mmol/g, 0.05 mmol/g to 1.2 mmol/g, 0.1 mmol/g to 1.2 mmol/g, 0.3 mmol/g to 1.2 mmol/g, 0.5 mmol/g to 1.2 mmol/g, 0.7 mmol/g to 1.2 mmol/g, 1.0 mmol/g to 1.2 mmol/g, 0.01 mmol/g to 1 mmol/g, 0.05 mmol/g to 1 mmol/g, 0.1 mmol/g to 1 mmol/g, 0.3 mmol/g to 1 mmol/g, 0.5 mmol/g to 1 mmol/g, or 0.7 mmol/g to 1 mmol/g, but is not limited thereto.

In an embodiment, an expansion coefficient per unit mass of the functional coating in water may be 2 mL/g to 8 mL/g. Specifically, the expansion coefficient per unit mass of the functional coating in water may be 2 mL/g to 8 mL/g, 2 mL/g to 7 mL/g, 2 mL/g to 6 mL/g, 2 mL/g to 5 mL/g, 3 mL/g to 8 mL/g, 3 mL/g to 7 mL/g, 3 mL/g to 6 mL/g, or 3 mL/g to 5 mL/g, but is not limited thereto. Furthermore, the functional coating causes swelling in a solvent other than water, and thus the expansion coefficient per unit mass may vary depending on the types of solvents.

In an embodiment, the core structure may have solvent resistance, thermal resistance, or both properties. Unlike the functional coating, the core structure does not exhibit a property of swelling or a property of dissolving in a solvent. Thus, it is capable of self-standing even at high flow velocity, and may serve as a support for the composite solid support. Furthermore, since the core structure has heat resistance, it may serve as a support without change even when biopolymer synthesis is carried out at high temperatures.

In an embodiment, components of the core structure may include at least one selected from a polymer, a metal, and ceramic. Specifically, the polymer may include at least one selected from high-density polyethylene (HDPE), low-density polyethylene (LDPE), linear low-density polyethylene (LLDPE), ultra-high-molecular-weight polyethylene (UHMWPE), polypropylene (PP), polyethylene terephthalate (PET), polybutylene terephthalate (PBT), polyethylene naphthalate (PEN), polyamide (PA), polyvinylchloride (PVC), polyvinyl difluoride (PVDF), polyacetal, polycarbonate (PC), polyimide (PI), polyetheretherketone (PEEK), polyethersulfone (PES), polyphenylene oxide (PPO), and polyphenylenesulfide (PPS), but is not limited thereto. The metal may include at least one selected from stainless steel, titanium, nickel, tantalum, zirconium, and an alloy thereof, but is not limited thereto. The ceramic may include at least one selected from fused silica, alumina, zirconia, silicon carbide, silicon nitride, boron nitride, and titanium diboride, but is not limited thereto.

In an embodiment, the core structure may have at least one shape selected from a one-dimensional shape shape, a two-dimensional shape, and a three-dimensional shape.

In an embodiment, the one-dimensional shape may include at least one selected from a staple fiber, a filament fiber, and a rod. As a specific example, the one-dimensional shape of the core structure may include a fiber shape, and in this case, the core structure may have a length sufficient to enable biopolymer synthesis. The composite solid support is loaded in a tangled form into the reactor, so as to be utilized in a biopolymer synthesis reaction.

In an embodiment, the two-dimensional shape may include at least one selected from a spunbond non-woven fabric, a meltblown non-woven fabric, a needle-punched non-woven fabric, a hydroentangled non-woven fabric, a woven fabric, a knitted fabric, a porous membrane, a polymeric film, and a mesh. As a specific example, the two-dimensional shape of the core structure may include a form in which a plurality of core structures in the one-dimensional shape are randomly arranged. In addition, as voids are formed among the plurality of core structures in the one-dimensional shape through the random arrangement, the two-dimensional shape may exhibit random porosity.

In an embodiment, the three-dimensional shape may include an open cell foam, a macro-pored sphere, or both shapes.

In an embodiment, the length of one cross-section of the core structure may be 1 mm or more, which is a size sufficient to involve the functional coating in the desired biopolymer synthesis. As a specific example, when the core structure has the one-dimensional shape, the core structure having a fiber shape or the like may become entangled to serve as a support of the composite solid support. In addition, when the core structure has the two-dimensional or three-dimensional shape, the core structure itself may serve as a support in the form of a non-woven fabric (two-dimensional) or a foam (three-dimensional). Therefore, the length of one cross-section of the core structure may be 1 mm or more, and theoretically, the core structure in the one-dimensional shape with infinite length may also serve as a support.

In an embodiment, the functional coating may include a polymeric product of one or more main monomers and an active monomer. As a specific example, the functional coating may include: a polymeric product of a first monomer and the active monomer; or a polymeric product of a first monomer, a second monomer, and the active monomer. In addition, the functional coating of the present disclosure may be a polymer formed through polymerization between the active monomer, which provides a reaction site, and one or more main monomers as main components.

In an embodiment, the first monomer may include at least one selected from a bisacrylamide-based crosslinking agent, a methacryloyl group, an alkenyl group-substituted triazine, and an acrylate-based crosslinking agent. Specifically, the first monomer may include a polyethylene glycol-based crosslinking agent such as polyethylene glycol diacrylate. Specifically, as the first monomer, any material may be used without limitation as long as it is capable of reacting with or bonding to the active monomer to expose the functional groups within and/or on the surface of the functional coating. As a non-limiting example, the first monomer may include at least one selected from polyethylene glycol diacrylate, N,N'-methylenebisacrylamide (MBA), ethylene glycol dimethacrylate (EGDMA), poly(ethylene glycol) dimethacrylate (PEGDMA), glycidyl methacrylate (GMA), divinyl sulfone (DVS), triethylene glycol divinyl ether (TEGDVE), and diallyl phthalate (DAP). The second monomer may include at least one selected from 2-hydroxyethyl acrylate, 2-hydroxyethyl methacrylate (HEMA), acrylic acid(AA), methyl methacrylate (MMA), ethyl acrylate (EA), butyl acrylate (BA), glycidyl methacrylate (GMA), and vinylacetate (VAc).

In an embodiment, the active monomer is to provide the functional groups within and/or on the surface of the functional coating, and may include at least one selected from an acrylate-based material, an acrylamide-based material, and a methacrylamide-based material. As a non-limiting example, the active monomer may include at least one selected from N-(2-aminopropyl) methacrylamide hydrochloride, aminoethyl methacrylate hydrochloride (AEMA.HCI), 2-aminoethyl Methacrylate (2-AEMA), N-(3-aminopropyl) methacrylamide hydrochloride (APMA), 4-aminostyrene), N-(4-aminophenyl)methacrylamide, N,N-dimethylaminoethyl methacrylate (DMAEMA), N,N-dimethylaminopropyl acrylamide (DMAPAA), and N-(2-aminoethyl)acrylamide hydrochloride.

In an embodiment, the composite solid support may include a homogeneous composite solid support, a heterogeneous composite solid support, or both composite solid supports. Specifically, in the homogeneous composite solid support, the functional coating may be a polymeric product of the active monomer and the one or more main monomers. In addition, in the heterogeneous composite solid support, the core structure may be coated with the functional coating including a pulverized conventional solid support. The conventional solid support may include at least one selected from polystyrene/divinylbenzene copolymer (PS/DVB), crosslinked polyethyleneglycol, poly-ε-lysine/sebacic acid, controlled pore glass, amino-polyacrylamide-resin fiber, cellulose, and hydroxylated PP, but is not limited thereto.

In an embodiment, the one-dimensional core structure may have a diameter (cross-section) of 10 µm to 100 µm. Specifically, the diameter of the one-dimensional core structure may be 10 µm to 100 µm, 20 µm to 100 µm, 30 µm to 100 µm, 40 µm to 100 µm, 10 µm to 80 µm, 20 µm to 80 µm, 30 µm to 80 µm, 40 µm to 80 µm, 10 µm to 60 µm, 20 µm to 60 µm, 30 µm to 60 µm, 40 µm to 60 µm, 10 µm to 50 µm, 20 µm to 50 µm, 30 µm to 50 µm, or 40 µm to 50 µm, but is not limited thereto. When the diameter of the one-dimensional core structure is less than 10 µm, the core structure becomes too thin, causing the core structure to agglomerate, thereby degrading mechanical properties. When the diameter of the one-dimensional core structure exceeds 100 µm, the loading density of the functional coating may become excessively low.

In an embodiment, the two-dimensional core structure may have a thickness (layer thickness) of 10 µm to 100 µm. Specifically, the thickness of the two-dimensional core structure may be 10 µm to 10 mm, 50 µm to 10 mm, 100 µm to 10 mm, 150 µm to 10 mm, 200 µm to 10 mm, 250 µm to 10 mm, 300 µm to 10 mm, 350 µm to 10 mm, 400 µm to 10 mm, 10 µm to 5 mm, 50 µm to 5 mm, 100 µm to 5 mm, 150 µm to 5 mm, 200 µm to 5 mm, 250 µm to 5 mm, 300 µm to 5 mm, 350 µm to 5 mm, 400 µm to 5 mm, 10 µm to 3 mm, 50 µm to 3 mm, 100 µm to 3 mm, 150 µm to 3 mm, 200 µm to 3 mm, 250 µm to 3 mm, 300 µm to 3 mm, 350 µm to 3 mm, 400 µm to 3 mm, 10 µm to 1 mm, 50 µm to 1 mm, 100 µm to 1 mm, 150 µm to 1 mm, 200 µm to 1 mm, 250 µm to 1 mm, 300 µm to 1 mm, 350 µm to 1 mm, 400 µm to 1 mm, 10 µm to 0.5 mm, 50 µm to 0.5 mm, 100 µm to 0.5 mm, 150 µm to 0.5 mm, 200 µm to 0.5 mm, 250 µm to 0.5 mm, 300 µm to 0.5 mm, 350 µm to 0.5 mm, or 400 µm to 0.5 mm, but is not limited thereto. When the thickness of the two-dimensional core structure is less than 10 µm, it becomes difficult to maintain mechanical properties, and when the thickness of the two-dimensional core structure exceeds 10 mm, there is a possibility that the polymerization will not occur uniformly to the inside of the functional coating.

In an embodiment, the core structure may have a porosity of 50 % to 95 %. In the case of the one-dimensional core structure, the core structure having a fiber shape or the like may form voids through entanglement, and in the case of the two-dimensional or three-dimensional core structure, the core structure may include voids therein, such as in a non-woven fabric or foam. In such cases where the core structure includes voids, the porosity of the core structure may be 50 % to 95 %, 60 % to 95 %, 70 % to 95 %, 80 % to 95 %, 90 % to 95 %, 50 % to 90 %, 60 % to 90 %, 70 % to 90 %, or 80 % to 90 %, but is not limited thereto. Here, the size of the porous separator structure is not particularly limited, and when the porosity is less than 50 %, the voids are clogged even after the functional coating swells, so that biopolymer synthesis may not occur to the inside of the functional coating. In addition, when the porosity exceeds 95 %, it may be difficult to maintain mechanical properties.

In an embodiment, the thickness of the functional coating may be 0.1 µm to 1000 µm. The thickness of the functional coating may be selected to an appropriate thickness depending on the types of desired biopolymers and based on whether the core structure has a one-dimensional shape, a two-dimensional shape, or a three-dimensional shape. Specifically, the thickness of the functional coating may be 0.1 µm to 1000 µm, 0.5 µm to 1000 µm, 1 µm to 1000 µm, 5 µm to 1000 µm, 10 µm to 1000 µm, 50 µm to 1000 µm, 100 µm to 1000 µm, 0.1 µm to 500 µm, 0.5 µm to 500 µm, 1 µm to 500 µm, 5 µm to 500 µm, 10 µm to 500 µm, 50 µm to 500 µm, 100 µm to 500 µm, 0.1 µm to 200 µm, 0.5 µm to 200 µm, 1 µm to 200 µm, 5 µm to 200 µm, 10 µm to 200 µm, 50 µm to 200 µm, 100 µm to 200 µm, 0.1 µm to 100 µm, 0.5 µm to 100 µm, 1 µm to 100 µm, 5 µm to 100 µm, 10 µm to 100 µm, 50 µm to 100 µm, 0.1 µm to 50 µm, 0.5 µm to 50 µm, 1 µm to 50 µm, 5 µm to 50 µm, 10 µm to 50 µm, 0.1 µm to 20 µm, 0.5 µm to 20 µm, 1 µm to 20 µm, 5 µm to 20 µm, or 10 µm to 20 µm, but is not limited thereto. When the thickness of the functional coating is excessively small, such as less than 0.1 µm, a problem occurs in which the loading density of the functional coating becomes very low. In addition, when the thickness of the functional coating is excessively large, such as greater than 1000 µm, the functional coating itself may be manufactured through uneven polymerization, potentially leading to reduced purity during a biopolymer synthesis process.

The composite solid support may not have a shell-core structure or a bead structure. In addition, the solid support may be core-free in a shell-core structure.

In an embodiment, the surface of the core structure may be hydrophilically treated. Specifically, such a hydrophilic treatment on the surface may be performed using a method selected from a chemical method utilizing a surfactant or an acidic solution and a physical method such as plasma treatment or UV irradiation, but is not limited thereto. The functional coating may be formed on the core structure having a hydrophilically treated surface.

In an embodiment, the functional coating may further include a linker.

In an embodiment, the linker may include at least one selected from a Rink Amide linker, a Wang linker, a 2-chlorotrityl (CTC) linker, a Sieber linker, a BAL linker, a 4-sulfamylbutyryl linker, and a HMBA (TFA stable) linker.

In an embodiment, the functional coating may have the functional groups exposed within and/or on surface of the functional coating, wherein the linker may be bonded to the functional groups. By adding additional monomer units (amino acid residues) to monomer units (amino acid residues) connected through the functional coating (functional group)-linker, a biopolymer including a plurality of monomer units may be synthesized.

In an embodiment, the biopolymer may include at least one selected from a peptide, an oligonucleotide, and a peptide nucleic acid (PNA), but is not limited thereto.

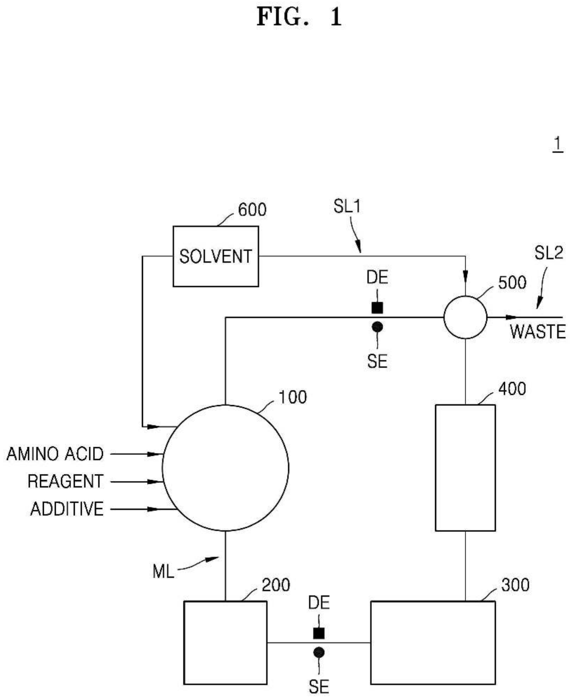

FIG. 1 is a diagram illustrating a biopolymer synthesis system according to an embodiment of the present disclosure.

Referring to FIG. 1, a biopolymer synthesis system 1 according to an embodiment of the present disclosure may enable the biopolymer synthesis by forming a circulating flow within a fluid containing at least one of an amino acid, a reagent, and an additive.

Hereinafter, 'amino acid mixture" is a liquid containing not only amino acid(s) used herein but also reagent(s), wherein the 'amino acids' may be modified or unmodified, and may optionally be pre-activated. The amino acid mixture may also include a peptide.

Hereinafter, 'SPPS' is an abbreviation for solid phase peptide synthesis, and is used to mean production of a peptide by adding an amino acid residue to a peptide or amino acid fixed on a solid support (resin).

Hereinafter, 'reagent' is used to mean a coupling reagent, a deprotecting reagent, an additive, a base, and other reagents used in synthesis.

Hereinafter, 'fluid' is defined as a substance discharged from a reservoir and flowing along piping of the biopolymer synthesis system, and depending on a process, the fluid may contain various types of single substances or mixed substances.

In an embodiment, the biopolymer synthesis system 1 may employ a flow-through process for SPPS that enables sequential synthesis of at least one amino acid.

In an embodiment, the biopolymer synthesis system 1 may include a mixing reservoir 100, a pump 200, a temperature controller 300, and a reactor 400. In addition, the biopolymer synthesis system 1 may further include a valve 500, a solvent reservoir 500, a sensor SE, and a detector DE.

A main line ML may provide a circulation piping line by connecting the mixing reservoir 100, the pump 200, the temperature controller 300, and the reactor 400 together. In addition, the valve 500 is arranged on the main line ML, and may be connected to both a first branch line SL1 and a second branch line SL2.

The mixing reservoir 100 may provide a space where a fluid containing at least one of an amino acid, a reagent, and an additive remains. The mixing reservoir 100 is provided with an opening/closing valve (not shown) arranged at an outlet end so that the type and flow rate of the fluid flowing through the main line ML may be controlled.

In the mixing reservoir 100, at least one of an amino acid, a reagent, and an additive provided from a reagent reservoir (not shown) and the solvent reservoir 600 may be mixed. By controlling an opening amount of each chamber, an operator may precisely control the type and flow rate of the fluid discharged from the mixing reservoir 100 for each synthesis stage. As an example, in each stage of the biopolymer synthesis by using the SPPS, the corresponding fluid may be introduced into the mixing reservoir 100, mixed and held therein, and then introduced into the reactor 400.
1) Deprotection stage: Deprotecting reagent (piperidine)
2) Washing stage: Solvent (DMF)
3) Coupling stage: Amino acid, coupling reagent (DIC), and additive (Oxyma pure)
4) Washing stage: Solvent (DMF)

The pump 200 provides a driving force to the fluid such that the fluid may flow along the main line ML. The pump 200 may provide a driving force to enable the fluid to flow from the mixing reservoir 100 to the reactor 400. Referring to FIG. 1, the pump 200 is positioned between the mixing reservoir 100 and the reactor 400 in consideration of the fuid flow. However, since the fluid forms a circulating flow, the pump 200 does not necessarily have to be positioned between the mixing reservoir 100 and the reactor 400.

The pump 200 may be any type of devices capable of providing the fluid with suction and discharge forces. For example, the pump 200 may be a gear pump, a screw pump, a vane pump, a cap pump, a piston pump, a plunger pump, a diaphragm pump, a centrifugal pump, or the like.

In addition, the pump 200 may be any type of pumps, including a mechanical displacement type micropump and an electromagnetic motion micropump. The mechanical displacement type micropump may be a pump, such as a gear or a diaphragm, utilizing the motion of solids or fluids to cause a pressure difference to induce a fluid flow, and may include a diaphragm displacement pump, a fluid displacement pump, a rotary pump, or the like. The electromagnetic motion micropump may be a pump directly utilizing electrical or magnetic energy to a fluid flow, and may include an electro-hydrodynamic pump (EHD), an electro-osmotic pump, a magneto-hydrodynamic pump, an electro-wetting pump, or the like.

The temperature controller 300 may set the temperature of the fluid flowing into the reactor 400 to a target temperature. The temperature controller 300 is positioned downstream of the mixing reservoir 100 and upstream of the reactor 400 to cool or heat the fluid containing at least one selected from an amino acid, a reagent, and an additive. Referring to FIG. 1, the temperature controller 300 is positioned between the mixing reservoir 100 and the reactor 400 in consideration of the fluid flow. However, since the fluid forms a circulating flow, the temperature controller 300 does not necessarily have to be positioned between the mixing reservoir 100 and the reactor 400.

The temperature controller 300 may be any device that transfers heat to the fluid or receives heat from the fluid, via heat exchange. For example, the temperature controller 300 may be an electric heating device, an induction heater, a microwave cavity, or the like.

As an example, the temperature controller 300 may set the temperature of the fluid flowing into the reactor 400 to a room temperature. The temperature controller 300 may control the temperature of the fluid to 120 °C or lower, specifically 25 °C to 100 °C. In addition, the temperature controller 300 may control the temperature of the fluid to 50 °C to 80 °C.

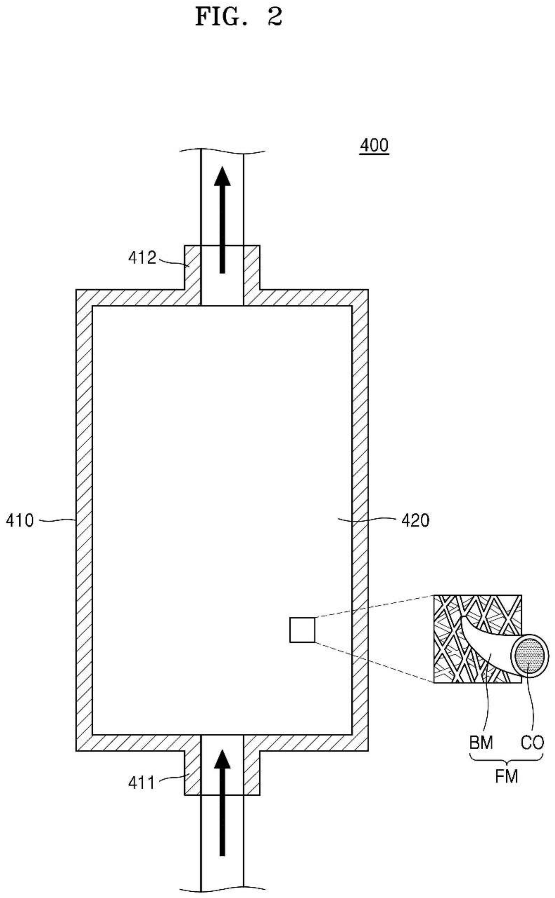

FIG. 2 is a drawing illustrating the reactor 400 in FIG. 1.

Referring to FIGS. 1 and 2, in the reactor 400, the fluid discharged from the mixing reservoir 100 may circulate and flow.

The reactor 400 may include a column 410 and a composite solid support 420.

The column 410 may have an inlet 411 through which the fluid enters and an outlet 412 through which the fluid flows out. The column 410 has an internal space, and the composite solid support 420 may be placed therein.

The size of the column 410 may vary depending on the type of biopolymers being synthesized, the amount of synthesized biopolymers, the flow rate of the fluid, the flow velocity of the fluid, the type of the composite solid support, or the like.

The inlet 411 and outlet 412 of the column 410 are arranged on the main line ML, such that the fluid entering through the inlet 411 may pass through the internal space of the column 410, and then may be discharged through the outlet 412. Here, the fluid may pass through the composite solid support 420 placed within the internal space of the column 410.

The composite solid support 420 may be placed inside the column 410. The solid support 420 may fill the inside of the column 410.

The composite solid support 420 may be arranged in various positions and shapes within the internal space of the column 410. The composite solid support 420 may fill the internal space of the column 410 at a predetermined position.

For example, the composite solid support 420 may be loaded into the internal space of the column 410.

As an example, when the core structure of the composite solid support 420 is in the form of entangled filaments FM, the fluid may pass between the filaments FM. A flow path of the fluid may vary depending on a loading method of the composite solid support 420. As an example, when the composite solid support 420 in a spirally wound shape is loaded, the fluid may flow through porous spaces created by entanglement between each layer of the spirally wound composite solid support 420.

The composite solid support 420 may include a core structure (also referred to as a base material (BM)) and a functional coating CO.

As an example, the core structure BM may form random porosity by having a plurality of filaments entangled. The functional coating CO may refer to coating of the core structure BM with a polymer for synthesizing a biopolymer, so as to expose the functional groups such as amine groups and carboxyl groups.

The core structure BM may have a length of 1 mm or more and a shape of one dimension or higher dimensions. The core structure FM may have at least one shape selected from a one-dimensional shape, a two-dimensional shape, and a three-dimensional shape.

The core structure BM may have solvent resistance, thermal resistance, or both properties. Unlike the functional coating CO, the core structure BM does not exhibit a property of swelling or a property of dissolving in a solvent. Thus, it is capable of self-standing, and may serve as a support for the composite solid support. Furthermore, since the core structure FM has heat resistance, it may serve as a support without change even when synthesis of a biopolymer is carried out at high temperatures.

The functional coating CO may surround the core structure FM and provide an area where a biopolymer is synthesized.

As an example, the functional coating CO may have a property of swelling in a solvent.

The functional coating CO may include functional groups within or on the surface of the functional coating CO. Specifically, the functional coating CO may include functional groups, and due to the property of swelling in a solvent, a reactant may easily penetrate into the functional coating CO by the concentration gradient. Accordingly, functional groups within or on the surface of the functional coating may be easily exposed to the reactant, exhibiting high synthesis efficiency even at low loading density.

The valve 500 is arranged between the reactor 400 and the reservoir 100, and may set a path for discharging waste. The valve 500 may control the flow direction of the fluid to discharge the waste generated after reactions in the reactor 400 to the second branch line SL2. As an example, the valve 500 may be a four-channel valve, and the four-channel valve may be connected to the mixing reservoir 100, the reactor 400, the solvent reservoir 600, and the second branch line SL2.

The valve 500 may control circulation of the fluid and discharge of the waste. The valve 500 may be used to set a circulating flow of reactant fluid during each stage of SPPS synthesis, thereby increasing synthesis efficiency of a biopolymer. The valve 500 may also assist progression to the next stage by discharging residual materials from each stage into the second branch line SL2.

The solvent reservoir 600 may store solvents used for synthesizing and cleaning a biopolymer. The solvent may be selected in various ways depending on a synthesis process.

The solvent reservoir 600 may be arranged on the first branch line SL1. The solvent reservoir 600 is connected to the first branch line SL1 so that the solvent may be transferred to the valve 500 or to the mixing reservoir 100 according to each stage of the SPPS synthesis.

Before performing a washing process during the synthesis process, any residual material remaining in the piping from the previous step must be removed. Here, the solvent discharged from the solvent reservoir 600 may be discharged through the first branch line SL1, so that the main line ML may be washed.

A sensor SE is arranged on the main line ML, and may sense at least one of a temperature, a pressure, and a flow rate of the fluid flowing along the main line ML.

A detector DE is arranged on the main line ML to detect the progress of biopolymer synthesis. As an example, the detector DE may be arranged at at least one of the inlet 411 and the outlet 412 of the reactor 400, and may detect the circulating fluid. In FIG. 1, the detector DE is positioned between the reactor 400 and the mixing reservoir 100 in consideration of the fluid flow, but since the fluid forms a circulating flow, the detector DE does not necessarily have to be positioned between the reactor 400 and the mixing reservoir 100.

The main line ML may connect the mixing reservoir 100, the pump 200, the temperature controller 300, and the reactor 400 together, thereby providing a circulating path of the fluid.

The first branch line SL1 branches off from the main line ML between the mixing reservoir 100 and the reactor 400, and thus may be connected to the mixing reservoir 100. The first branch line SL1 connects the valve 500 and the mixing reservoir 100, supplying the solvent from the solvent reservoir 600 to the main line ML to remove the residual materials from the main line ML.

The second branch line SL2 branches off from the valve 500, and may discharge the waste.

Hereinafter, preferable examples are presented to help understanding of the present disclosure. However, the following examples are only presented for easier understanding of the present disclosure, and the contents of the present disclosure are not limited by the following examples.

### Example 1. Analysis of flow velocity-dependent pressure drop in reactor

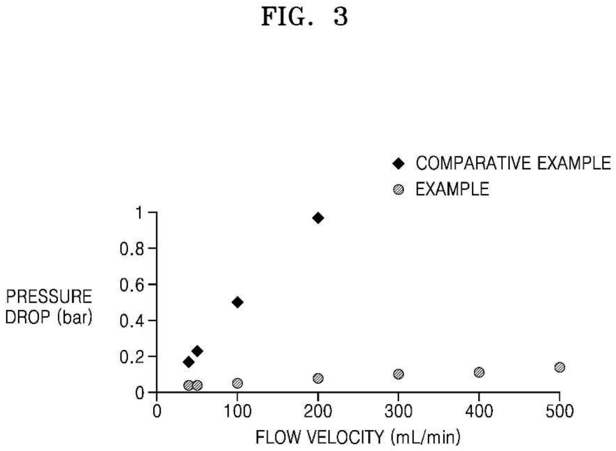

FIG. 3 is a graph measuring flow velocity-dependent pressure drop to compare the reactor of the present disclosure with a reactor of a comparative example.

### (Test devices)

- Column (Intertec empty cartridge column): 27 mL, I.D./Height: 12.8*60 mm
- Pump: LEAD FLUID CT3001, pump head: Fluid-o-Tech/MG209
- Reagents used: Dimethylformaminde (DMF, SAMJHUN), Rink-amide-MBHA-resin (GL Biochme, 0.5 mmole/g)
- Pressure measurement device: SMC PSE560-C01 pressure sensor, SMC PSE200A digital pressure sensor controller

As an example, 5.0 g (0.5 mmol, loading density: 0.1 mmol/g) of a composite solid support was placed in a column, and as a comparative example, 1.0 g (0.5 mmol, loading density: 0.5 mmol/g) of a particulate solid support (PS/DVB) was placed in a column. Then, 20 mL of DMF solvent was added to each column to allow swelling of the composite solid support for 30 minutes.

Subsequently, a discharge tube of a pump was connected to the column, pressure sensors were each installed at an inlet and an outlet of the column, and each pressure sensor was connected to a controller.

The pressures at the inlet and outlet were measured while flowing DMF at a flow velocity of 40 mL/min (1.48 C.V/min) into an empty column, a composite solid support column (Example), and a particulate solid support column (Comparative Example). Afterwards, the pressures at the inlet and outlet of the column were measured by changing the flow velocity to 50 ml/min (1.85 C.V/min), 100 ml/min (3.7 C.V/min), 200 ml/min (7.4 C.V/min), 300 ml/min (11.11 C.V/min), 400 ml/min (14.81 C.V/min), or 500 ml/min (18.52 C.V/min).

Values of pressure drop occurring at the inlet and outlet of the empty column were set as reference values (zero). Values of additional pressure drop occurring at the inlet and outlet of the column including the composite solid support were calculated based on the reference values, and values of additional pressure drop occurring at the inlet and outlet of the column including the particulate composite solid support were calculated based on the reference values.

**[Table 1]**

| Flow velocity (ml/min) | Comparative Example | Example |
|---|---|---|
| 40 | 0.17 | 0.04 |
| 50 | 0.23 | 0.04 |
| 100 | 0.5 | 0.05 |
| 200 | 0.97 | 0.08 |
| 300 | - | 0.1 |
| 400 | - | 0.11 |

Referring to FIG. 3 and Table 1, the composite solid support of the present disclosure showed a small pressure drop even when the flow velocity increased. That is, since the fluid was able to pass through the empty space of the composite solid support, even when the flow velocity increased, only a small pressure was resulted and the flowability of the fluid increased. On the other hand, the conventional particulate solid support (bead-type solid-phase synthetic resin polymer (PS/DVB)) showed a large pressure drop when the flow velocity increased, and no fluid was discharged at a flow velocity of 300 ml/min. In other words, the conventional particulate solid support caused particles to agglomerate when the flow velocity increased, filling the space within the column and reducing the space available for fluid passage. Consequently, the pressure measured at the outlet significantly dropped, and the flowability of the fluid decreased.

### Example 2. Analysis of pressure drop of composite solid supports according to column size

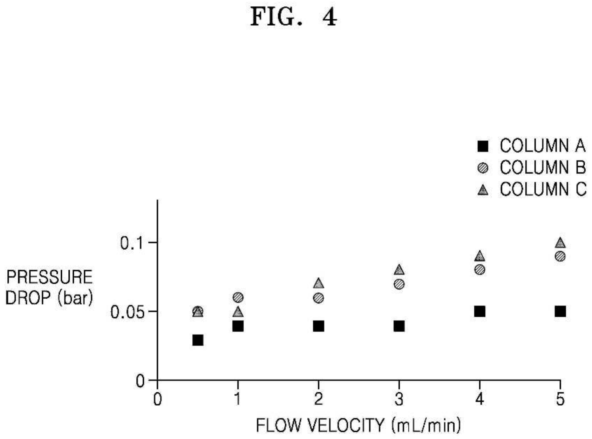

FIG. 4 is a graph measuring pressure drop according to the size of the reactor of the present disclosure.

### (Test devices)

- Column A (Intertec empty cartridge column): 27 mL, I.D./Height: 12.8*60 mm
- Column B (Intertec empty cartridge column): 108 mL, I.D./Height: 21.4*76 mm)
- Column C (Intertec empty cartridge column): 385 mL, I.D./Height:26.8*127 mm
- Pump: LEAD FLUID CT3001, pump head: Fluid-o-Tech/MG209
- Reagents used: Dimethylformaminde (DMF, SAMJHUN), Rink-amide-MBHA-resin (GL Biochme, 0.5 mmole/g)
- Pressure measurement device: SMC PSE560-C01 pressure sensor, SMC PSE200A digital pressure sensor controller

Column A was loaded with 5 g of the composite solid support, 20 ml of DMF solvent was added, and the mixture was allowed to swell for 30 minutes. Column B was loaded with 20 g of the composite solid support, 80 ml of DMF solvent was added, and the mixture was allowed to swell for 30 minutes. Column C was loaded with 70 g of the composite solid support, 300 ml of DMF solvent was added, and the mixture was allowed to swell for 30 minutes.

A discharge tube of the pump was connected to each column, pressure sensors were each installed at an inlet and outlet of each column, and each pressure sensor was connected to a controller.

The pressures at the inlet and outlet were measured while flowing DMF into each of Column A, Column B, and Column C at a flow velocity of 0.5 column volume (mL)/min. Afterwards, the pressures at the inlet and outlet of each column were measured by changing the flow velocity to 1 C.V/min, 2 C.V/min, 3 C.V/min, 4 C.V/min, or 5 C.V/min.

Values of pressure drop occurring at the inlet and outlet of each empty column were set as reference values (zero). Values of additional pressure drop occurring at the inlet and outlet of each of Columns A, B, and C containing the composite solid support compared to the reference values were calculated.

**[Table 2]**

| Flow velocity (C.V/min) | Column A | Column B | Column C |
|---|---|---|---|
| 0.5 | 0.03 | 0.05 | 0.05 |
| 1 | 0.04 | 0.06 | 0.05 |
| 2 | 0.04 | 0.06 | 0.07 |
| 3 | 0.04 | 0.07 | 0.08 |
| 4 | 0.05 | 0.08 | 0.09 |

Referring to FIG. 4 and Table 2, the composite solid support of the present disclosure showed a small pressure drop in the fluid at various flow velocities, even when the volume of the column was varied. The reason for this is that the fluid could pass through the empty space in the composite solid support, resulting in a small pressure drop and increased flowability of the fluid even when the flow velocity increased. Accordingly, the composite solid support of the present disclosure had a consistently small degree of pressure drop regardless of the volume of the column.

### Example 3. Analysis of pressure drop of composite solid supports according to temperature

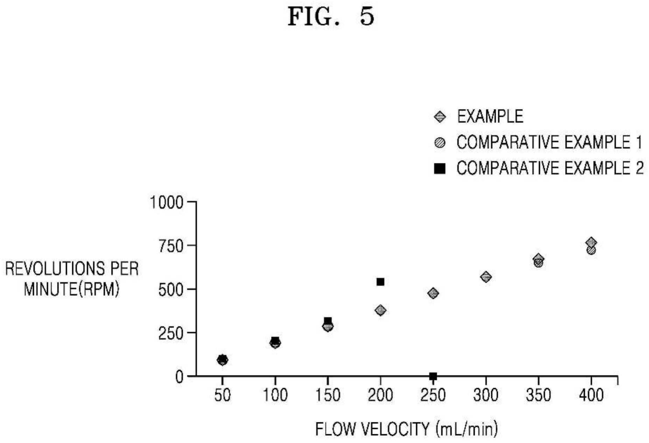

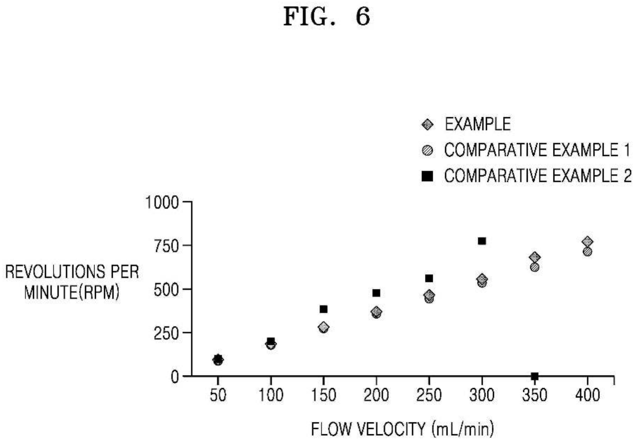

FIGS. 5 and 6 are graphs measuring temperature-dependent pressure drop in the reactor of the present disclosure and a reactor of a comparative example.

At the same flow velocity, the back pressure is high when the rotational speed is high. In this regard, when the fluid was discharged at a specific flow rate, the rotational speed of the pump was measured to compare the back pressure applied to each solid support.

A 27 ml column loaded with the composite solid support (0.5 mmol) used in the present disclosure (Example), an empty column (Comparative Example 1), and a 27 ml column loaded with a conventional particulate solid support (Rink-Amide-MBHA-resin, 0.5 mmol, 1.25 g) (Comparative Example 2) were compared.

As shown in Table 3 and FIG. 5, the RPM values of the pump were recorded at the flow velocity of 50, 100, 150, 200, 250, 300, 350, and 400 ml/min under conditions of using a DMF solvent at 25 °C.

When DMF was used at 25 °C, the column loaded with the composite solid support (Example) did not show a significant difference in the rotational speed of the pump from the empty column (Comparative Example 1). On the other hand, the column loaded with the particulate solid support (Comparative Example 2) stopped the operation of the pump at 250 ml/min due to high back pressure.

**[Table 3]**

| Flow velocity (ml/min) | Examples | Comparative Example 1 | Comparative Example 2 |
|---|---|---|---|
| 50 | 96 | 94 | 103 |
| 100 | 191 | 188 | 205 |
| 150 | 287 | 282 | 317 |
| 200 | 383 | 376 | 542 |
| 250 | 479 | 471 | 0 |
| 300 | 574 | 565 | 0 |
| 350 | 670 | 646 | 0 |
| 400 | 766 | 724 | 0 |

As shown in Table 4 and FIG. 6, the RPM values of the pump were recorded at the flow velocity of 50, 100, 150, 200, 250, 300, 350, and 400 ml/min under conditions of using a DMF solvent at 50 °C. When using DMF at 50 °C, the viscosity of the solution decreased, resulting in a decrease in the back pressure. However, the particulate solid support (Comparative Example 2) stopped the operation of the pump at 350 ml/min due to high back pressure.

**[Table 4]**

| Flow velocity (ml/min) | Examples | Comparative Example 1 | Comparative Example 2 |
|---|---|---|---|
| 50 | 92 | 89 | 97 |
| 100 | 184 | 177 | 194 |
| 150 | 276 | 266 | 378 |
| 200 | 368 | 355 | 474 |
| 250 | 459 | 443 | 554 |
| 300 | 551 | 532 | 768 |
| 350 | 669 | 621 | 0 |
| 400 | 765 | 710 | - |

As shown in Table 5, the RPM values of the pump were recorded at the flow velocity of 50, 100, 150, 200, 250, 300, 350, and 400 ml/min under conditions of using a DMF solvent at 70 °C. When using DMF at 70 °C, the viscosity of the solution decreased, resulting in a decrease in the back pressure. However, the particulate solid support (Comparative Example 2) stopped the operation of the pump at 350 ml/min due to high back pressure.

**[Table 5]**

| Flow velocity (ml/min) | Examples | Comparative Example 1 | Comparative Example 2 |
|---|---|---|---|
| 50 | 89 | 87 | 94 |
| 100 | 180 | 175 | 189 |
| 150 | 271 | 259 | 375 |
| 200 | 364 | 349 | 465 |
| 250 | 453 | 440 | 543 |
| 300 | 547 | 530 | 730 |
| 350 | 659 | 618 | 0 |
| 400 | 763 | 705 | - |

In addition, the revolutions per minute of the pump was measured at room temperature by using piperidine 20 % in DMF (0.1 M Oxymapure), which is widely used in the SPPS. Likewise, the particulate solid support stopped the operation at 250 ml/min due to high back pressure. That is, it was confirmed that the composite solid support of the present disclosure involved less back pressure than the conventional particulate solid support. Considering that synthesizing longer peptides increases the volume and weight of the composite solid support, requiring greater back pressure, the composite solid support of the present disclosure, unlike the conventional particulate solid support, may enhance the flowability of the fluid to improve the synthesis performance of a flow reactor.

The flow reactor and the biopolymer synthesis system including the same, according to an embodiment of the present disclosure, may therefore increase the synthesis efficiency.

Conventional particulate resin polymers for solid-phase synthesis agglomerate at high flow velocity and fill the internal space of the column without any empty spaces, thereby reducing the flowability of the reactor. Thus, a large pressure drop occurred at the outlet of the reaction, resulting in a high back pressure. Such a high back pressure will not significantly scale up the synthesis of the synthesis system.

The flow reactor and the biopolymer synthesis system including the same, according to an embodiment of the present disclosure, may increase flowability of the reactor by including the composite solid support. Thus, the reactor may have a low pressure drop, and subsequently a low back pressure, enabling an increase in the synthesis scale.

Specifically, under the same pressure conditions, a low back pressure results in a small decrease (loss) in flow velocity, thereby promoting mixing and diffusion between the reactants and the reaction solution within the column. In this regard, the biopolymer synthesis system may have improved flow dynamics with increased flow velocity of the fluid, thereby improving the reaction efficiency and yield.

In addition, a decreased back pressure may also reduce wear and mechanical stress on the internal components of the reactor, thereby improving the durability of the entire system. In addition, pumps capable of providing various pressures may be applied, thereby increasing usability and range of options available. In particular, even when using a low-pressure pump, high reaction efficiency and yield may be exhibited.

In addition, a reduced back pressure may reduce the risk of damage to piping and connections through which the fluid flows, reducing leakage of the fluid. Accordingly, the durability of the system may be improved as well as the process stability and reliability.

In addition, when the back pressure is reduced, the reaction of the fluid passing through the reactor may be precisely controlled, thereby improving reproducibility of the reaction process and enabling biopolymer synthesis with consistent quality and high quality.

### Example 4. Manufacture of composite solid support

### (1) Manufacture of polymer for functional coating

By using the previously qualified flow reactor, an experiment was carried out to measure the performance of synthesizing a biopolymer. First, a polymer for functional coating used in a composite solid support was manufactured. Reagents shown in Table 6 were used, and a solution in which each reagent was mixed and stirred in a solvent to dissolve uniformly was prepared. Here, components of the polymer for functional coating only need to satisfy the range of respective mass ratio (parts by weight), and the solvent may be used regardless of the mass ratio since it will be dried later. In the experiment, the sum of respective reagent and solvent (D.W) was measured to be 100 g [wherein the solvent may be used within a range of 10 wt% to 90 wt% of the total (reagent+solvent)].

**[Table 6]**

| Classificatio n | Name of reagent | Parts by mass |
|---|---|---|
| First monomer | Polyethylene glycol diacrylate | 100 |
| Second monomer | Methyl acrylate | 0 to 50 |
| Active monomer | N-(2-aminopropyl) methacrylamide hydrochloride | 1 to 25 |
| Surfactant | Sodium dodecyl sulfate | 0 to 25 |

Afterwards, 0.5 g of 2-hydroxy-2-methylpropiophenone, a curing initiator, was added to the solution, and then mixed uniformly to prepare a functional coating solution. The functional coating solution was uniformly applied to a petri dish and sufficiently irradiated with ultraviolet energy of 10 mJ/cm² to 1,000 mJ/cm² to ensure curing. After completion of the curing, the reaction residues were removed using ethanol to obtain a functional coating polymer disk. The mass of the dried polymer for functional coating was measured to be 45.7 g. The functional coating polymer thus manufactured was quick-frozen using liquid nitrogen, and then pulverized using a mortar. Subsequently, using 100- and 200-mesh sieves, polymer particles for functional coating with a particle size range of 100 to 200 mesh (74 to 149 µm) were obtained.

### (2) Manufacture of composite solid support for biopolymer synthesis

To manufacture a composite solid support, the functional coating solution (before undergoing the curing) in (1) of Example 4 and 20 g of a core structure (polypropylene, spunbond nonwoven fabric, 40 g/m²) were prepared. After sufficiently impregnating the core structure with the functional coating solution and removing the core structure from the functional coating solution, the resulting core structure was sufficiently irradiated with ultraviolet energy of 10 mJ/cm² to 1,000 mJ/cm² in an ultraviolet curing chamber.

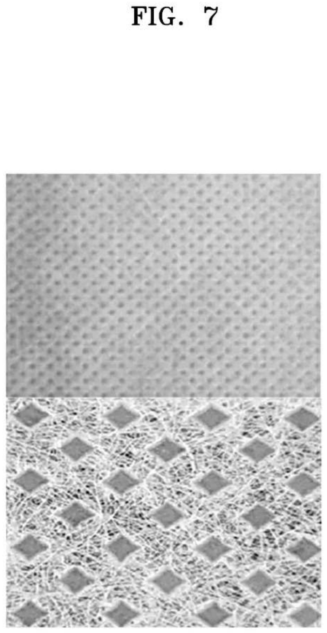

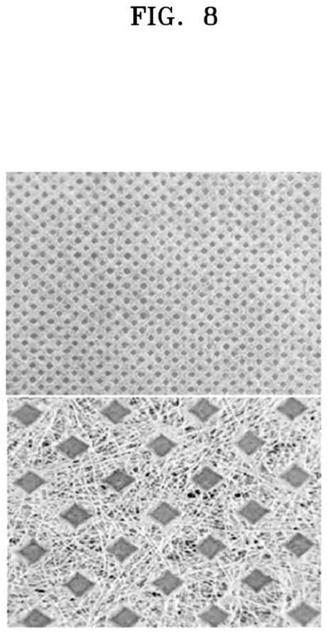

After completion of the curing, the coated core structure was subjected to removal of the residuals using ethanol, followed by drying. A composite solid support including the core structure coated with the polymer for functional coating was obtained (solid support 30.4 g, 60.8 g/m²). The appearance of the core structure before undergoing the coating is as shown in FIG. 7, and the appearance of the composite solid support is as shown in FIG. 8.

### Example 5. Synthesis of biopolymer by using composite solid support

In comparison with a method using a batch-type automatic synthesizer (CEM Liberty, USA), it was confirmed whether synthesis of peptides was possible by SPPS using a biopolymer synthesis system including a column-type flow reactor containing a solid support for biopolymer synthesis according to an embodiment, and whether there was a difference in the purity and yield of synthesized peptides.

### (1) Synthesis of peptide by using batch-type automatic synthesizer

After adding 0.1 mmol of each of the existing particulate solid support for solid-phase synthesis and the composite solid support according to an embodiment to an automatic synthesizer, a synthesis reaction of acyl carrier protein (ACP) model peptide (65-74) of SEQ ID NO: 1 was carried out under the same conditions as in Table 7.

SEQ ID NO: 1: VQAAIDYING

**[Table 7]**

| | | | | |
|---|---|---|---|---|
| Reactio n stage | Reagent (solvent: DMF) | Volume (mL) | Temperatu re (°C) | Time (minutes) |

| | | | | |
|---|---|---|---|---|
| Deprot ection | 20 % piperidine,0.1 M oxyma pure | 15 | 70 | 2 |
| Washin g | DMF | 10 (3 times) | - | - |
| Couplin g | Fmoc-Ile (0.2 M) | 6 | 70 | 4 |
| | DIC (0.5 M) | 2 | | |
| | Oxyma pure (1.0 M) | 1 | | |
| Washin g | DMF | 10 (2 times) | - | - |

The particulate solid support and the composite solid support that have completely undergone the peptide synthesis reaction were each thoroughly washed with ethanol and dried, and then subjected to a cleavage reaction using a cleavage solution (95 % TFA, 2.5 % TSI, 2.5 % DW). Afterwards, following precipitation using cold ether, solids were obtained by centrifugation washed twice more using 15 ml of cold ether, and then sufficiently dried using a drying method under reduced pressure. Using high-performance liquid chromatography (HPLC), the purity and yield of dried peptides were measured. The results of measuring the purity and yield of dried peptides are shown in Table 8.

**[Table 8]**

| Types of solid support | Purity (%) | Yield (%) |
|---|---|---|
| Composite solid support | 85.1 | 88.4 |
| Particulate solid support | 81.4 | 71.5 |

According to the analysis of peptide synthesis using the existing commercial batch-type SPPS synthesizer (Liberty, CEM, USA) for the particulate solid support and the biopolymer composite solid support according to an embodiment, the ACP peptides showed some improvement in terms of the yield and purity in the composite solid support according to an embodiment, as compared to the particulate solid support.

### (2) Synthesis of peptide by using flow-based column synthesizer

To compare the synthesis performance of a particulate solid support and a homogeneous composite solid support in a flow-type reactor, 0.5 mmol of the particulate solid support and 0.5 mmol of the composite solid support were added to a 27 mL column separately, and under the conditions shown in Table 9, reaction processes were carried out by changing the types of amino acids, thereby synthesizing an ACP model peptide (65-74) of SEQ ID NO: 1. Afterwards, the dry mass was measured to calculate the yield, and the purity of the synthesized peptide was measured by HPLC.

**[Table 9]**

| Reaction stage | Reagent (solvent: DMF) | Volume (mL) | Tempe rature (°C) | Time (minut es) | Flow rate (mL/min) |
|---|---|---|---|---|---|
| Deprotec tion | 20 % piperidine, 0.1 M oxyma pure | 25 | 70 | 2 | |
| Washing | DMF | 125 | - | - | |
| Coupling | Fmoc-amino acid (0.2 M) | 15 | 70 | 4 | 100 |
| | DIC (0.5 M) | 6 | | | |
| | Oxyma pure (1.0 M) | 3 | | | |
| Washing | DMF | 20 | - | - | |

The synthesis performance in the flow-type reactor using the composite solid support was shown to be superior to that in a batch-type reactor in terms of both synthesis purity and yield. In contrast, when the ACP(65-74) peptide synthesis was carried out by filling the flow-type reactor with the particulate solid support, a column blocking problem (e.g., backpressure issue) occurs during the synthesis, and thus the synthesis was not performed properly. The reason for this is that the particulate solid support blocks the column of the flow-type reactor due to swelling. However, the composite solid support in which the core structure is coated with the functional coating has no potential for such issues to arise, and thus is more suitable for use in the flow-type reactor. Accordingly, it was confirmed that the composite solid support was suitable for a batch-type synthesis system and a flow-type synthesis system compared to the existing particulate solid support. In particular, when the composite solid support was applied to a flow-type synthesis system, the yield and purity of the ACP peptide were found to be 90 % or more, demonstrating significantly excellent efficacy in the biopolymer synthesis.

### Example 6. Analysis and evaluation of yield and purity changes of synthesized peptide according to flow rate of biopolymer in flow reactor

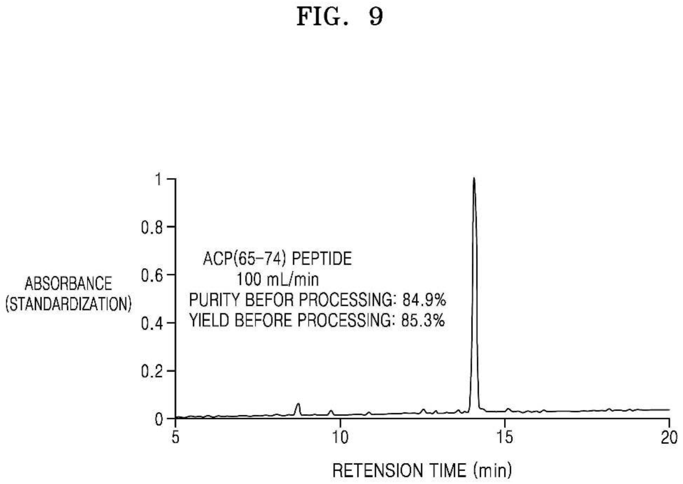

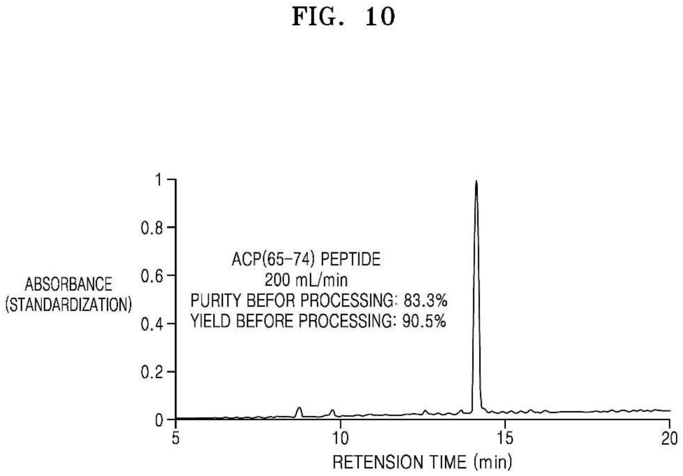

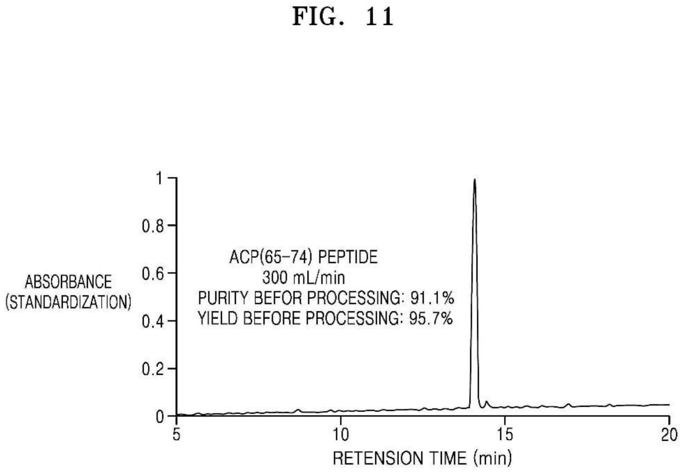

As in Example 5, the yield and purity of the synthesized peptide according to the flow rate were confirmed using the composite solid support. The ACP was synthesized using the same method as in Example 5, and the results for the purity and yield of the synthesized ACP peptide are shown in Table 10 and FIGS. 9 to 11.

**[Table 10]**

| Flow rate (mL/min) | Yield (%) | Purity (%) |
|---|---|---|
| 100 | 84.9 | 85.3 |
| 200 | 88.3 | 90.5 |
| 300 | 91.1 | 95.7 |

FIGS. 9 to 11 show HPLC graphs for the ACP synthesized according to the flow rate, by using the composite solid support for biopolymer synthesis according to an embodiment. When using the solid support for biopolymer synthesis according to an embodiment, it was confirmed that increasing the flow rate of the column synthesizer resulted in improved purity and yield of the peptide.

The results indicate that a flow-based reactor including a column is a more suitable reactor for the composite solid support according to an embodiment.

The present disclosure has thus been described with reference to the embodiments shown in the drawings, but these embodiments are only exemplary, and those skilled in the art can understand that various modifications and other equally valid embodiments are possible therefrom. Therefore, the true technical protection scope of the present disclosure should be determined by the technical idea of the appended claims.

## Claims

1. A biopolymer synthesis system comprising:
a mixing reservoir in which at least one of an amino acid, a reagent, and an additive is mixed;
a reactor into which a fluid discharged from the mixing reservoir is introduced;
a temperature controller arranged between the mixing reservoir and the reactor and configured to control a temperature of the fluid introduced into the reactor; and
a pump configured to provide a driving force such that the fluid flows from the mixing reservoir to the reactor,
wherein the reactor comprises:
a column comprising an inlet through which the fluid enters and an outlet through which the fluid flows out; and
a composite solid support arranged inside the column.

2. The biopolymer synthesis system of claim 1, wherein
the composite solid support comprises:
a core structure; and
a functional coating positioned on the core structure.

3. The biopolymer synthesis system of claim 2, wherein the composite solid support is self-standing when the fluid passes through the column.

4. The biopolymer synthesis system of claim 2, wherein, in the composite solid support, the functional coating has a loading density of 0.01 mmol/g to 2 mmol/g.

5. The biopolymer synthesis system of claim 1, wherein, when the fluid passes through the reactor, a pressure drop at the inlet and the outlet is 10 bar or less at a flow velocity of 40 C.V/min or less.

6. The biopolymer synthesis system of claim 1, wherein a temperature of the fluid that has passed through the temperature controller and subsequently flows into the reactor is 120 °C or lower.

7. The biopolymer synthesis system of claim 1, further comprising a valve arranged between the reactor and the mixing reservoir and configured to discharge waste.

8. The biopolymer synthesis system of claim 1, further comprising
a main line connecting the mixing reservoir, the pump, the temperature controller, and the reactor together; and
a first branch line branching off from the main line between the mixing reservoir and the reactor and being connected to the mixing reservoir.

9. The biopolymer synthesis system of claim 8, wherein the fluid has a circulating flow through the main line.

10. The biopolymer synthesis system of claim 8, further comprising a solvent reservoir arranged on the first branch line.

11. The biopolymer synthesis system of claim 10, wherein the solvent reservoir is connected to the valve and the mixing reservoir through the first branch line.

12. The biopolymer synthesis system of claim 8, further comprising a sensor arranged on the main line and configured to sense at least one of a temperature, a pressure, and a flow rate of the fluid flowing along the main line.

13. The biopolymer synthesis system of claim 8, further comprising a detector arranged on the main line and configured to detect progress of biopolymer synthesis.

14. The biopolymer synthesis system of claim 7, further comprising a second branch line branching off from the valve to discharge the waste.
